# EUROPÄISCHE PATENTANMELDUNG

(11) **EP 4 623 946 A1**
(43) Veröffentlichungstag der Anmeldung: **01.10.2025**
(21) Anmeldenummer: 25165793.8
(22) Anmeldetag: 24.03.2025
(51) Int. Cl.: A61M 1/16

(54) **SYSTEM UMFASSEND EINE VORRICHTUNG ZUR EXTRAKORPORALEN BLUTBEHANDLUNG UND EINEN WÄRMETAUSCHER**

(30) Priorität: 25.03.2024 DE 102024108454
(71) Anmelder: B. Braun Avitum AG, 34212 Melsungen (DE)
(72) Erfinder: JANIK, Waldemar, 34212 Melsungen (DE); LINDNER, Joshua, 34212 Melsungen (DE)
(74) Vertreter: Maiwald GmbH

(57) **Zusammenfassung**

Die Erfindung betrifft ein System umfassend eine Vorrichtung zur extrakorporalen Blutbehandlung und einen Wärmetauscher zum Wärmeaustausch zwischen von der Vorrichtung zur extrakorporalen Blutbehandlung abfließendem Dialysat und der Vorrichtung zur extrakorporalen Blutbehandlung zuzuführendem Permeat, wobei die Vorrichtung zur extrakorporalen Blutbehandlung und der Wärmetauscher als separate Einrichtungen ausgebildet sind.

## Beschreibung

### TECHNISCHES GEBIET

Die Erfindung betrifft ein System umfassend eine Vorrichtung zur extrakorporalen Blutbehandlung, beispielsweise eine Dialysemaschine, und einen Wärmetauscher zum Wärmeaustausch zwischen Dialysat und Permeat und einen Wärmetauscher zur Verwendung in einem solchen System.

### HINTERGRUND DER ERFINDUNG

Bei Dialysesystemen ist in der Regel der Wärmetauscher integral mit der Vorrichtung zur extrakorporalen Blutbehandlung, beispielsweise Dialysemaschine, ausgebildet, was in der Praxis bedeutet, dass diese in ein gemeinsames Fluidsystem verbaut sind und häufig auch durch ein gemeinsames Gehäuse eingehaust sind. Es gibt jedoch recht unterschiedliche Einsatzszenarien für solche Vorrichtungen, beispielsweise Dialysemaschinen, und die bekannten Lösungen können den gewünschten Einsatzszenarien nicht immer gerecht werden, was sich beispielsweise in reduzierter Effizienz äußern kann.

Eine Aufgabe, die der Erfindung zugrunde liegt, ist eine System zur Verfügung zu stellen, das einer größeren Bandbreite an Einsatzszenarien gerecht wird.

### ZUSAMMENFASSUNG DER ERFINDUNG

Die vorliegende Erfindung stellt ein System gemäß Anspruch 1 bereit. Bevorzugte Ausführungsformen sind in den abhängigen Ansprüchen beschrieben. Die Erfindung betrifft auch einen Wärmetauscher zur Verwendung in einem solchen System.

Das System gemäß der vorliegenden Offenbarung umfasst eine Vorrichtung zur extrakorporalen Blutbehandlung, insbesondere Dialysemaschine, und einen Wärmetauscher zum Wärmeaustausch zwischen von der Vorrichtung zur extrakorporalen Blutbehandlung abfließendem Dialysat und der Vorrichtung zur extrakorporalen Blutbehandlung zuzuführendem Permeat, wobei die Vorrichtung zur extrakorporalen Blutbehandlung und der Wärmetauscher als separate Einrichtungen ausgebildet sind.

In der nachfolgenden Beschreibung werden zur besseren Lesbarkeit die Merkmale und Erläuterungen, stellvertretend für die "Vorrichtung zur extrakorporalen Blutbehandlung" am Beispiel einer "Dialysemaschine" beschrieben, durch das alle Vorrichtungen zur extrakorporalen Blutbehandlung miterfasst sein sollen, beispielsweise neben der Dialysemaschine auch Vorrichtungen zur Hämofiltration, Hämodiafiltration, Blutbegasung oder Apherese.

Der Wärmetauscher kann beispielsweise in der Hämodialyse und/oder der Peritonealdialyse eingesetzt werden.

Mit anderen Worten wird ein System bereitgestellt, das einen bezüglich der Dialysemaschine externen Wärmetauscher umfasst. Der Wärmetauscher kann von der Dialysemaschine, insbesondere strukturell, derart unabhängig sein, dass er ohne Umbau an der Dialysemaschine und/oder am Wärmetauscher und/oder ohne Öffnen der Dialysemaschine mit der Dialysemaschine verbindbar und von der Dialysemaschine trennbar ist. Der Wärmetauscher kann von der Dialysemaschine, insbesondere strukturell, derart unabhängig sein, dass er gleichzeitig oder nacheinander ohne Umbau der Dialysemaschine mit mehreren Dialysemaschinen verwendbar ist.

Als Permeat kann die frische Lösung in Strömungsrichtung im Normalbetrieb vor dem Dialysator bezeichnet werden, insbesondere vor dem Zumischen von Konzentraten zum Erzeugen der Dialysierflüssigkeit. Vor dem Erreichen des Dialysators werden im Normalbetrieb üblicherweise ein oder mehrere Konzentrate dem Permeat zugemischt, in der Regel ein basisches und ein saures Konzentrat. Die so entstandene Mischung wird als Dialysierflüssigkeit bzw. Dialysierlösung bezeichnet und diese wird dem Dialysator zugeführt. Als Dialysat kann die verbrauchte Lösung nach Durchlaufen des Dialysators bezeichnet werden. Das Dialysat ist im ordnungsgemäßen Betrieb in der Regel wärmer als das Permeat und kann, mittels des Wärmetauschers, das Permeat vorwärmen, so dass die Wärme des Dialysats noch nutzbringend verwendet werden kann.

Derzeit wird der Wärmetauscher integral mit der Dialysemaschine ausgebildet, was in der Praxis bedeutet, dass diese in ein gemeinsames Fluidsystem verbaut und häufig durch ein gemeinsames Gehäuse eingehaust sind.

Das System gemäß der vorliegenden Offenbarung sieht abweichend davon vor, die Dialysemaschine und den Wärmetauscher als separate Einrichtungen auszubilden.

Dies kann eine hohe Effizienz des Systems für verschiedene Bedarfsfälle ermöglichen. So kann beispielsweise die Dimensionierung des Wärmetauschers bedarfsgerecht gewählt werden. Komponenten des Systems können flexibel verwendet werden, um optimalen Betrieb sicher zu stellen. So können beispielsweise mehrere Wärmetauscher für eine Dialysemaschine vorgesehen sein oder ein Wärmetauscher für mehrere Dialysemaschinen. Mehrere Wärmetauscher können unabhängig voneinander oder in Reihe geschaltet verwendet werden. Wärmetauscher können auch leicht ergänzt, entfernt, oder ausgetauscht und/oder die Schaltung der Komponenten (Wärmetauscher und Dialysemaschine(n)) flexibel an den Bedarfsfall angepasst werden. So kann durch die erhöhte Flexibilität für eine Vielzahl von Bedarfsfällen eine hohe Effizienz gewährleistet werden. Es kann also ein System zur Verfügung gestellt werden, das einer größeren Bandbreite an Einsatzszenarien gerecht wird.

Gemäß der vorliegenden Offenbarung können die Dialysemaschine und der Wärmetauscher über einen ersten Anschluss der Dialysemaschine, einen zweiten Anschluss der Dialysemaschine, einen ersten Anschluss des Wärmetauschers und einen zweiten Anschluss des Wärmetauschers miteinander verbunden sein. Das System kann optional eine erste Leitung, beispielsweise einen ersten Schlauch, und eine zweite Leitung, beispielsweise einen zweiten Schlauch, umfassen. Der erste Anschluss des Wärmetauschers kann mittels der ersten Leitung mit dem ersten Anschluss der Dialysemaschine verbunden sein und der zweite Anschluss des Wärmetauschers kann mittels der zweiten Leitung mit dem zweiten Anschluss der Dialysemaschine verbunden sein. Optional können die erste Leitung und/oder die zweite Leitung wärmeisoliert sein.

Das heißt, die Dialysemaschine und der Wärmetauscher können über die jeweiligen Anschlüsse trennbar miteinander verbunden sein. Die Verbindung kann eine unmittelbare Verbindung sein, sofern die Anschlüsse miteinander kompatibel sind, oder eine mittelbare Verbindung, beispielsweise über einen Adapter oder, wie oben beschrieben, über Leitungen, wobei jeweils zwei Anschlüsse über eine Leitung miteinander verbunden sind. So kann das Dialysesystem einfach und flexibel konfiguriert werden. Durch entsprechende zusätzliche Anschlüsse an der Dialysemaschine und/oder an dem Wärmetauscher und/oder durch entsprechend ausgebildete Leitungsstücke und/oder Schaltelement kann zudem ermöglicht werden, mehrere Dialysemaschinen an einen Wärmetauscher anzuschließen und/oder mehrere Wärmetauscher an eine Dialysemaschine anzuschließen. Somit kann, angepasst an das jeweilige Nutzungsszenario, eine passende Konfiguration hergestellt werden, was hohe Effizienz für verschiedene Nutzungsszenarien ermöglicht. Eine optionale Wärmeisolation der Leitungen ermöglicht, diese Flexibilität und Effizienz zu erreichen und zugleich die Wärmeverluste gering zu halten, selbst bei längeren Leitungen bzw. größeren Abständen.

Der Wärmetauscher kann einen Wärmeübertragungsabschnitt, mindestens ein mittels eines Aktors schaltbares Ventil und einen Sensor umfassen, insbesondere einen Temperatursensor. Der Wärmetauscher kann dazu ausgebildet sein, das Ventil mittels des Aktors basierend auf Sensordaten des Sensors zu schalten. Insbesondere kann der Wärmetauscher dazu ausgebildet sein, derart zu schalten, dass bei Feststellen eines Überschreitens eines ersten Temperaturschwellwerts einer von der Dialysemaschine durch den Wärmetauscher abfließenden Flüssigkeit, beispielsweise des Dialysats oder einer bei einer Desinfektion, Entkalkung, und/oder Reinigung der Dialysemaschine verwendeten Flüssigkeit, mittels des Sensors das mindestens eine Ventil derart geschaltet wird, dass die abfließende Flüssigkeit und/oder das Permeat nicht durch den Wärmeübertragungsabschnitt fließt.

Das Vorsehen eines solchen Ventils kann ermöglichen, eine Umleitung der Strömung von Permeat und/oder der abfließenden Flüssigkeit derart vorzunehmen, dass kein Wärmeaustausch zwischen Permeat und der abfließenden Flüssigkeit stattfindet. Insbesondere kann beispielsweise das Permeat umgeleitet werden, so dass es nicht durch den Wärmeübertragungsabschnitt fließt.

Das oben beschriebene Schalten ermöglicht beispielsweise auch Wärmenutzung der Wärme der abfließenden Flüssigkeit für andere Prozesse. Das Koppeln an das Überschreiten eines Temperaturschwellwerts kann ein Sicherheitsmerkmal sein, das ermöglicht, zu verhindern, dass das Permeat durch die abfließende Flüssigkeit zu sehr erwärmt werden würde, und/oder kann ermöglichen, sehr hohe Temperaturen der abfließenden Flüssigkeit günstig zu nutzen, beispielsweise für Prozesse, die eine höhere Temperatur benötigen, beispielsweise für Reinigungsprozesse.

Der Wärmetauscher kann einen/den Temperatursensor umfassen und der Wärmetauscher kann dazu ausgebildet sein, bei Überschreiten eines zweiten Temperaturschwellwerts einer von der Dialysemaschine durch den Wärmetauscher abfließenden Flüssigkeit, beispielsweise des Dialysats oder einer bei einer Desinfektion, Entkalkung, und/oder Reinigung der Dialysemaschine verwendeten Flüssigkeit, automatisch eine Wartungsflüssigkeit, beispielsweise Reinigungsmittel, Desinfektionsmittel oder Entkalkungsmittel, in Strömungswege des Wärmetauschers zuzuführen, insbesondere in einen/den Wärmeübertragungsabschnitt des Wärmetauschers.

Reinigungsprozesse haben in der Regel bei höheren Temperaturen eine bessere Wirksamkeit. Erkennen und Nutzen einer ausreichend hohen Temperatur der abfließenden Flüssigkeit können alternativ zu einem dedizierten Heizen zu Reinigungszwecken eingesetzt werden oder können ein solches Heizen unterstützen. Das erhöht wiederum die Effizienz des Systems.

Der Wärmetauscher kann einen Wartungsflüssigkeitszufuhrabschnitt aufweisen, über den eine/die Wartungsflüssigkeit, beispielsweise Reinigungsmittel, Desinfektionsmittel oder Entkalkungsmittel, in Strömungswege des Wärmetauschers zuführbar ist, insbesondere in einen/den Wärmeübertragungsabschnitt des Wärmetauschers.

Insbesondere kann das System derart ausgebildet sein, dass das oben beschriebene automatische Zuführen der Reinigungsflüssigkeit ein automatisches Herstellen einer Fluidverbindung zwischen dem Wartungsflüssigkeitszufuhrabschnitt und den Strömungswegen, insbesondere dem Wärmeübertragungsabschnitt, bei Überschreiten eines zweiten Temperaturschwellwerts umfassen. Dier kann anders, insbesondere höher als der erste Temperaturschwellwert sein. Beispielsweise kann das System ausgebildet sein, zum Herstellen der Fluidverbindung ein Ventil automatisch zu schalten.

Das System kann mehrere Dialysemaschinen umfassen und der Wärmetauscher mit den Dialysemaschinen über deren jeweiligen ersten Anschluss und zweiten Anschluss verbunden sein. Dazu kann der Wärmetauscher mehrere erste und zweite Anschlüsse umfassen, die jeweils mit den ersten und zweiten Anschlüssen der Dialysemaschinen verbunden sind. Alternativ oder zusätzlich können ein erster und ein zweiter Anschluss des Wärmetauschers jeweils mit mehreren ersten bzw. zweiten Anschlüssen der Dialysemaschinen verbunden sein. Das System kann entsprechende Schaltelemente und/oder Leitungsstücke umfassen.

Eine solche Verbindung ermöglicht, dass sich mehrere Dialysemaschinen einen Wärmetauscher teilen. So kann eine bessere Nutzung des Wärmetauschers und/oder eine gleichmäßigere Versorgung der Dialysemaschinen ermöglicht werden. Auch kann so eine bedarfsgerechte Versorgung ermöglicht werden. So kann insgesamt die Effizienz gesteigert werden. Auch ermöglicht dies größere Flexibilität hinsichtlich der Skalierung des Gesamtsystems.

Das System kann mehrere der Wärmetauscher umfassen. Dies ermöglicht größere Flexibilität hinsichtlich der Skalierung des Gesamtsystems. Außerdem kann ein Maß an Redundanz bereitgestellt werden, das ermöglicht, störungs-, reinigungs- oder wartungsbedinge Ausfälle abzufangen. Insbesondere kann das System mehrere bezogen auf die Strömungsrichtung in Reihe geschaltete Wärmetauscher umfassen.

Gemäß der vorliegenden Offenbarung kann der Wärmetauscher als Gegenstromwärmetauscher ausgebildet sein. Dies ist im Zusammenhang mit Dialysesystemen aufgrund der üblichen Konfiguration und des üblichen Betriebs besonders vorteilhaft.

Gemäß der vorliegenden Offenbarung kann der Wärmetauscher als Doppelrohrrekuperator, Rohrbündelrekuperator oder Plattenrekuperator ausgebildet sein.

Der Wärmeübertragungsabschnitt des Wärmetauschers kann aus Edelstahl oder polymerbasiertem Material, insbesondere Polypropylen oder Polyphenylensulfid, ausgebildet sein. Edelstahl weist hohe Wärmeleitfähigkeit und Robustheit auf, wohingegen polymerbasiertes Material leichter und somit transportabler ist und, beispielsweise durch additive Fertigungsverfahren, in vielfältigen Gestalten ausgebildet werden kann, beispielsweise um die Austauschfläche und/oder das Strömungsverhalten zu optimieren.

Der Wärmetauscher kann im ordnungsgemäßen Betrieb auf dem Boden stehen. Ein selbststehender Wärmetauscher kann ermöglichen, den Wärmetauscher beliebig zu dimensionieren, was beispielsweise bei einer hängenden Konfiguration schwieriger sein kann. Auch muss dann nicht auf Kompatibilität mit der Auslegung der Dialysemaschine geachtet werden.

Der Wärmetauscher kann Rollen aufweisen, mittels derer der Wärmetauscher transportierbar ist, insbesondere auf denen der Wärmetauscher beim Transport und optional im ordnungsgemäßen Betrieb gelagert ist. Die ermöglicht, auch bei größer dimensionierten Wärmetauschern, diesen flexibel so zu positionieren, wie es für den Betrieb geeignet ist, was insbesondere für flexible Systemkonfiguration (beispielsweise Zusammenschließen mehrerer Wärmetauscher und/oder Anschluss an eine oder mehrere Dialysemaschinen) vorteilhaft ist. Auch kann die einfache Positionierung Wärmeverlusten vorbeugen, weil dadurch der Weg zur Dialysemaschine gegebenenfalls durch neue Positionierung leicht reduziert werden kann.

Das System kann ein Hängesystem umfassen, das zum Aufhängen des Wärmetauschers an der Dialysemaschine, insbesondere an einem Maschinengehäuse der Dialysemaschine, ausgebildet ist.

Dies ermöglicht einen kurzen Strömungsweg zwischen Dialysemaschine und Wärmetauscher und somit auch geringere Wärmeverluste. Auch kann so die Mobilität des Systems erhöht werden, weil ein gemeinsames Bewegen vereinfacht wird. So können einige Vorteile integrierter Wärmetauscher auch für den externen Wärmetauscher erreicht werden.

Der Wärmetauscher kann, bezogen auf die Zulaufrichtung von Permeat, stromaufwärts eines Zulaufventils zum Dialysierflüssigkeitskreislaufs der Dialysemaschine angeordnet sein. Diese Anordnung ermöglicht ein flexibles Zuschalten zu und Wegschalten von der Dialysemaschine, ist also vorteilhaft für eine flexible Konfiguration des Systems, beispielsweise durch leichten Austausch des Wärmetauschers ohne Eingriff in den Dialysierflüssigkeitskreislauf der Dialysemaschine.

Der Dialysierflüssigkeitskreislauf kann beispielsweise die komplette Hydraulik zwischen Permeatzulauf der Dialysemaschine und Dialysatablauf umfassen. Beispielsweise kann der Dialysierflüssigkeitskreislauf eine Wasseraufbereitung in der Dialysemaschine, einen Entgasungsabschnitt für die Entgasung des Wassers, eine Dialysierflüssigkeitsaufbereitung, Abschnitte und/oder Einreichungen zur Förderung durch den Dialysator, Abschnitte und/oder Einrichtungen zur Bilanzierung von Dialysierflüssigkeit und Dialysat und/oder Abschnitte und Einrichtungen zur Förderung zum Abfluss umfassen. Insbesondere kann der Dialysierflüssigkeitskreislauf den Dialysator umfassen, sofern einer angebracht sein sollte. Der Wärmetauscher kann also beispielsweise als vor Beginn des (inneren) Hydrauliksystems der Dialysemaschine angeordnet betrachtet werden.

Der Wärmetauscher kann, bezogen auf die Zulaufrichtung von Permeat, zwischen einem Ringleitungssystem und der Dialysemaschine angeordnet sein, insbesondere wobei das System ein Ringleitungssystem umfasst und die Dialysemaschine im ordnungsgemäßen Betrieb über den Wärmetauscher mit dem Ringleitungssystem verbunden ist.

Dabei kann das Ringleitungssystem beispielsweise eine Wasserleitung, die von einer Wasseraufbereitungsanlage (beispielsweise Umkehrosmoseanlage) kommt und im Betrieb Dialysemaschinen mit Frischwasser versorgt, umfassen. Unverbrauchtes Wasser kann im Betrieb über das Ringleitungssystem der Umkehrosmoseanlage wieder zugeführt werden.

Die besagte Anordnung des Wärmetauschers ermöglicht besonders große Flexibilität hinsichtlich der Systemkonfiguration. Wäre beispielsweise der Wärmetauscher in das Ringleitungssystem integriert, wären manche der oben genannten flexiblen Gestaltungs- und Konfigurationsmöglichkeiten, insbesondere hinsichtlich Austauschs, Dimensionierung und flexibler Verschaltung möglicherweise schwieriger umsetzbar.

Die Dialysemaschine kann, wie oben schon erläutert, ein Maschinengehäuse aufweisen. An diesem, insbesondere an dessen Außenseite, können ein/der erste Anschluss und ein/der zweite Anschluss der Dialysemaschine angeordnet sein.

Diese Gestaltung ermöglicht, die Dialysemaschine abzuschirmen und dennoch eine einfache Möglichkeit bereitzustellen, Wärmetauscher fluidisch mit der Dialysemaschine zu verbinden. So kann insbesondere ohne Eingriffe in den Dialysierflüssigkeitskreislauf der Dialysemaschine, genauer ohne Eingriff in die Dialysemaschine, ein Wärmetauscher flexibel angeschlossen werden.

Der Wärmetauscher kann ein Gehäuse aufweisen. An diesem, insbesondere an dessen Außenseite, können ein/der erste Anschluss und ein/der zweite Anschluss des Wärmetauschers angeordnet sein.

Ein solches Gehäuse ermöglicht eine Abschirmung, insbesondere auch eine Wärmeabschirmung, bei zugleich einfacher Möglichkeit, den Wärmetauscher flexibel anzuschließen.

Die Dialysemaschine kann das Maschinengehäuse aufweisen und der Wärmetauscher außerhalb des Maschinengehäuses angeordnet sein. Insbesondere kann der gesamte Wärmetauscher vollständig außerhalb des Maschinengehäuses angeordnet sein.

Der Wärmetauscher kann das oben beschriebene Gehäuse aufweisen und die Dialysemaschine kann das Maschinengehäuse aufweisen, wobei das Gehäuse des Wärmetauschers außerhalb des Maschinengehäuses der Dialysemaschine angeordnet ist.

Die obigen Merkmale sind besonders vorteilhaft hinsichtlich der Flexibilität, da die Dialysemaschine und der Wärmetauscher voneinander unabhängig abgeschirmt und bewegbar sein können und das System somit flexibel konfigurierbar ist.

Der Wärmetauscher kann an die Dialysemaschine derart angeschlossen sein, dass im ordnungsgemäßen Betrieb im Wärmetauscher, insbesondere in einem/dem Wärmeübertragungsabschnitt des Wärmetauschers, ein Wärmeaustausch zwischen vom Wärmetauscher an die Dialysemaschine bereitzustellenden Permeat und aus der Dialysemaschine abfließenden Dialysat erfolgt. Insbesondere kann der Wärmetauscher entsprechende feste Strömungswege oder mittels Ventilen schaltbare Strömungswege umfassen. Der Wärmetausch kann durch Leiten der Strömung der Flüssigkeiten in entsprechende Strömungswege ermöglicht werden.

Das System, insbesondere der Wärmetauscher, kann eine Anzeigeeinheit aufweisen, die zum Anzeigen einer Ventilstellung eines/des Ventils und/oder von Sensordaten eines/des Sensors, insbesondere einer Temperatur einer von der Dialysemaschine abfließenden Flüssigkeit, insbesondere des Dialysats, ausgebildet ist. Beispielsweise kann eine Anzeigeeinheit, beispielsweise ein Bildschirm, an einem Gehäuse des Wärmetauschers angeordnet oder in das Gehäuse des Wärmetauschers integriert sein. Mittels einer solchen Anzeigeeinheit kann beispielsweise an einen Benutzer der Zustand des Systems, insbesondere des Wärmetauschers, ausgegeben werden, was dem Benutzer beispielsweise auch ein Eingreifen in den Betrieb ermöglichen kann.

Das System, insbesondere der Wärmetauscher, kann eine Energieversorgung, insbesondere eine Batterie und/oder Netzstromversorgung, zum Betreiben des Sensors und/oder zum Auslesen des Sensors und/oder zum Betreiben der Anzeigeeinheit und/oder zum Steuern eines Aktors zum Schalten des Ventils und/oder zum Betreiben des Aktors aufweisen.

Der Wärmetauscher kann alternativ oder zusätzlich einen thermoelektrischen Generator aufweisen, der dazu ausgebildet ist, mittels der Temperaturdifferenz zwischen einer von der Dialysemaschine abfließenden Flüssigkeit, insbesondere dem Dialysat, und Permeat Energie zum Betreiben des Sensors und/oder zum Auslesen des Sensors und/oder zum Betreiben der Anzeigeeinheit und/oder zum Steuern eines Aktors zum Schalten des Ventils und/oder zum Betreiben des Aktors und/oder zum Laden der Batterie bereitzustellen. Der Generator kann vom Seebeck- oder Peltier-Effekt Gebrauch machen.

Dies ermöglicht zumindest zeitweise autonomen Betrieb des Wärmetauschers. Auch kann es bei überschüssiger Wärme ermöglichen, diese noch nutzbringend zu verwenden. So kann die Effizienz weiter erhöht werden.

Das System kann eine Datenverbindung zwischen dem Wärmetauscher und der Dialysemaschine aufweisen und dazu ausgebildet sein, Sensordaten, insbesondere Daten eines Temperatursensors, von dem Wärmetauscher an die Dialysemaschine zu übertragen, insbesondere zum Regeln der Dialysierflüssigkeitstemperatur.

Optional können die Datenverbindung und die Energieversorgung mittels eines gemeinsamen Verbindungselements bereitgestellt sein, beispielsweise mittels eines Verbindungskabels, das Energieversorgung und Datentransfer erlaubt.

Dialysemaschinen enthalten in der Regel einen oder mehrere Temperatursensoren, deren Messwerte bei der Regelung des Betriebs der Dialysemaschine verwendet werden. Diese Regelung wird verbessert, wenn auch Temperaturdaten vom Wärmetauscher berücksichtigt werden. Beispielsweise kann bei der Herstellung von Dialysierflüssigkeit, die innerhalb einer Dialysemaschine stattfindet, neben der Zusammensetzung der Flüssigkeit auch ihre Temperatur angepasst werden. Die Temperaturregelung ist dabei oft ein komplexer Vorgang. Dies liegt insbesondere daran, dass die Regelstrecke meist recht groß ist, d.h. zwischen der Heizvorrichtung und dem Ort, wo die Solltemperatur vorliegen soll, können sich viele weitere Komponenten wie Ventile, Kammern, Abzweigungen, Filter und Sensoren befinden. Meist befinden sich über die Regelstrecke verteilt mehrere Temperatursensoren, deren Daten in eine Regelung der Dialysierflüssigkeitstemperatur eingehen. Gemäß der vorliegenden Offenbarung können auch die Temperatursensordaten vom Wärmetauscher in dieser Regelung berücksichtigt werden.

Daten, die von der Dialysemaschine an den Wärmetauscher bereitgestellt werden, können zur Schaltung von Ventilen des Wärmetauschers verwendet werden, beispielsweise für das oben beschriebene Ventil, das derart geschaltet werden kann, dass das Permeat und/oder das Dialysat nicht durch den Wärmeübertragungsabschnitt fließt. Beispielsweise kann es erforderlich sein, wenn der Temperatur-Zielwert (Solltemperatur) geringer als die momentane Temperatur der Dialysierflüssigkeit ist, zum Abkühlen eine Heizung abzuschalten und das warme Dialysat in den Abfluss zu fördern. In einem solchen Fall könnte im Wärmetauscher das Permeat ungewollt erwärmt werden. Durch einen Informationsaustausch der Solltemperatur von Dialysemaschine zu Wärmetauscher, kann das Ventil des Wärmetauschers, beispielsweise wie oben beschrieben, geöffnet werden, um einen Wärmeaustausch zu verhindern. Das Abkühlen der Hydraulik der Dialysemaschine kann dadurch effizienter gestaltet werden.

Die vorliegende Offenbarung betrifft auch ein Verfahren zum Wärmeaustausch zwischen von einer Dialysemaschine abfließendem Dialysat und der Dialysemaschine zuzuführendem Permeat mittels eines Wärmetauschers, wobei die Dialysemaschine und der Wärmetauscher als separate Einrichtungen eines Systems ausgebildet sind, insbesondere des Systems der vorliegenden Offenbarung. Die vorliegende Offenbarung betrifft auch eine Verwendung des Systems gemäß der vorliegenden Offenbarung zum Wärmetausch zwischen von der Dialysemaschine abfließendem Dialysat und der Dialysemaschine zuzuführendem Permeat. Die oben beschriebenen Vorteile und Merkmale gelten analog.

Die vorliegende Offenbarung betrifft auch einen Wärmetauscher zur Verwendung in einem System gemäß der vorliegenden Offenbarung, insbesondere zum Verbinden mit einer oder mehreren Dialysemaschinen gemäß der vorliegenden Offenbarung.

Insbesondere kann der Wärmetauscher wie oben im Zusammenhang mit dem System beschrieben ausgebildet sein.

### KURZE BESCHREIBUNG DER FIGUREN

Weitere Beispiele und Ausführungsformen werden im Folgenden anhand der Figuren erläutert. Dabei zeigen:
Figur 1 eine schematische und nicht-maßstabsgetreue Darstellung eines Systems gemäß der vorliegenden Offenbarung.
Figur 2 eine schematische und nicht-maßstabsgetreue Darstellung eines Systems gemäß der vorliegenden Offenbarung.
Figur 3 eine schematische und nicht-maßstabsgetreue Darstellung eines Wärmetauschers eines Systems gemäß der vorliegenden Offenbarung.
Figur 4 eine schematische und nicht-maßstabsgetreue Darstellung eines Wärmetauschers eines Systems gemäß der vorliegenden Offenbarung.

### DETAILLIERTE BESCHREIBUNG DER ERFINDUNG

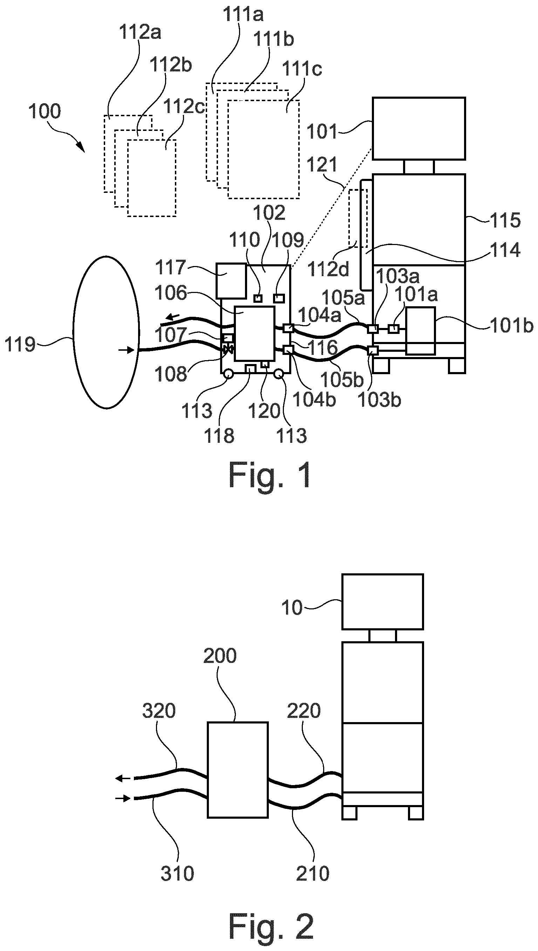

In Figur 1 ist ein System 100 gezeigt, das eine Vorrichtung zur extrakorporalen Blutbehandlung 101, beispielsweise eine Dialysemaschine, und einen Wärmetauscher 102 zum Wärmeaustausch zwischen von der Dialysemaschine abfließendem Dialysat und der Dialysemaschine zuzuführendem Permeat umfasst. Die Dialysemaschine und der Wärmetauscher sind als separate Einrichtungen ausgebildet. Beispielsweise kann es sich bei dem Wärmetauscher um einen Gegenstromwärmetauscher handeln. Es kommen verschiedene Arten von Wärmetauschern in Betracht, beispielsweise ein Doppelrohrrekuperator, ein Rohrbündelrekuperator oder ein Plattenrekuperator.

Der Wärmetauscher kann an die Dialysemaschine derart angeschlossen ist, dass im ordnungsgemäßen Betrieb im Wärmetauscher, insbesondere in einem Wärmeübertragungsabschnitt des Wärmetauschers, ein Wärmeaustausch zwischen vom Wärmetauscher an die Dialysemaschine bereitzustellenden Permeat und aus der Dialysemaschine abfließenden Dialysat erfolgt.

Gezeigt sind beispielhaft ein erster Anschluss 103a und ein zweiter Anschluss 103b der Dialysemaschine und ein erster Anschluss 104a und 104b des Wärmetauschers. Über die Anschlüsse sind der Wärmetauscher und die Dialysemaschine miteinander verbunden. Genauer können die ersten Anschlüsse 103a und 104a mittels einer ersten Leitung 105a miteinander verbunden sein, die beispielsweise als Schlauch ausgebildet sein kann. Die zweiten Anschlüsse 103b und 104b können mittels einer zweiten Leitung 105b miteinander verbunden sein, die beispielsweise als Schlauch ausgebildet sein kann. Die Leitungen können optional wärmeisoliert sein.

Der Wärmetauscher kann beispielsweise, bezogen auf die Zulaufrichtung von Permeat, stromaufwärts eines Zulaufventils 101a zum Dialysierflüssigkeitskreislaufs 101b der Dialysemaschine angeordnet sein.

In Figur 1 ist ein beispielhafter Wärmeübertragungsabschnitt 106 des Wärmetauschers gezeigt. Dieser kann beispielhaft aus Edelstahl oder polymerbasiertem Material, insbesondere Polypropylen oder Polyphenylensulfid ausgebildet sein.

Optional können, wie in Figur 1 gezeigt, ein Aktor 107, ein mittels des Aktors schaltbares Ventil 108 und ein Sensor 109 vorgesehen sein, die beispielsweise Teil des Wärmetauschers sein können. Bei dem Sensor kann es sich beispielsweise um einen Temperatursensor handeln.

Der Wärmetauscher kann beispielsweise dazu ausgebildet dein, das Ventil mittels des Aktors basierend auf Sensordaten des Sensors zu schalten, insbesondere derart zu schalten, dass bei Feststellen eines Überschreitens eines ersten Temperaturschwellwerts des Dialysats mittels des Sensors das Ventil derart geschaltet wird, dass das Permeat nicht durch den Wärmeübertragungsabschnitt fließt. Das heißt, das Permeat wird nicht mehr weiter mittels Wärmeübertragung erwärmt, wenn eine zu hohe Temperatur des Dialysats vorliegt. Alternativ kann beispielsweise das Permeat immer durch den Wärmeübertragungsabschnitt strömen und das Dialysat bei Bedarf an diesem Abschnitt vorbeigeleitet werden.

Der Wärmetauscher kann dazu ausgebildet sein, bei Überschreiten eines zweiten Temperaturschwellwerts des Dialysats automatisch eine Wartungsflüssigkeit, beispielsweise Reinigungsmittel, Desinfektionsmittel oder Entkalkungsmittel, in Strömungswege des Wärmetauschers zuzuführen, insbesondere in den Wärmeübertragungsabschnitt 106 des Wärmetauschers. Dies kann beispielsweise unter Verwendung von Messwerten des Sensors 109 erfolgen.

Der Wärmetauscher kann optional einen Wartungsflüssigkeitszufuhrabschnitt 110 aufweisen, über den Wartungsflüssigkeit, beispielsweise Reinigungsmittel, Desinfektionsmittel oder Entkalkungsmittel, in Strömungswege des Wärmetauschers zuführbar ist, insbesondere in den Wärmeübertragungsabschnitt 106 des Wärmetauschers.

Das System kann optional mehrere Dialysemaschinen umfassen. In Figur 1 sind beispielhaft in gestrichelten Linien optionale weitere Dialysemaschinen 111a bis 111c gezeigt. Der Wärmetauscher ist mit den Dialysemaschinen über deren jeweiligen ersten Anschluss und zweiten Anschluss verbunden. Dass hier drei weitere Dialysemaschinen gezeigt sind, ist rein beispielhaft zu verstehen, es können auch weniger oder mehr Dialysemaschinen vorgesehen sein.

Das System kann (alternativ oder zusätzlich zu etwaigen zusätzlichen Dialysemaschinen) zusätzliche Wärmetauscher 112a bis 112d umfassen, die in Figur 1 beispielhaft in gestrichelten Linien gezeigt sind. Diese können jeweils wie der Wärmetauscher 102 ausgebildet sein. Insbesondere können zumindest manche der Wärmetauscher bezogen auf die Strömungsrichtung in Reihe geschaltet sein. Dass hier vier weitere Wärmetauscher gezeigt sind, ist rein beispielhaft zu verstehen, es können auch weniger oder mehr Wärmetauscher vorgesehen sein.

Der Wärmetauscher kann im ordnungsgemäßen Betrieb auf dem Boden stehen, wie in der Figur 1 für den Wärmetauscher 102 gezeigt. Der Wärmetauscher kann optional Rollen 113 aufweisen, mittels derer der Wärmetauscher transportierbar ist, insbesondere auf denen der Wärmetauscher beim Transport und optional im ordnungsgemäßen Betrieb gelagert ist.

Wie am optionalen Wärmetaucher 112d gezeigt, kann der Wärmetauscher auch an der Dialysemaschine aufgehängt sein. Dafür kann das System ein Hängesystem 114 aufweisen, mittels derer der Wärmetauscher an der Dialysemaschine aufgehängt ist. Insbesondere kann der Wärmetauscher an einem Maschinengehäuse 115 der Dialysemaschine aufgehängt sein.

Der erste und der zweite Anschluss 103a und 103b der Dialysemaschine können an der Außenseite des Maschinengehäuses angeordnet sein. Der erste und der zweite Anschluss 104a und 104b des Wärmetauschers können an der Außenseite eines Gehäuses 116 des Wärmetauschers angeordnet sein.

In Figur 1 ist der gesamte Wärmetauscher, insbesondere samt Gehäuse 116, vollständig außerhalb des Maschinengehäuses 115 der Dialysemaschine angeordnet dargestellt. Dies ist eine beispielhafte Ausprägung des Merkmals, dass die Dialysemaschine und der Wärmetauscher separate Einrichtungen sind.

Das System, insbesondere der Wärmetauscher, kann eine Anzeigeeinheit 117 aufweisen, die zum Anzeigen einer Ventilstellung des Ventils 108 und/oder von Sensordaten des Sensors 109, insbesondere einer Temperatur des Dialysats, ausgebildet ist.

Das System, insbesondere der Wärmetauscher, kann eine Energieversorgung 118, insbesondere eine Batterie und/oder Netzstromversorgung, zum Betreiben des Sensors und/oder zum Auslesen des Sensors und/oder zum Betreiben der Anzeigeeinheit und/oder zum Steuern des Aktors 107 zum Schalten des Ventils und/oder zum Betreiben des Aktors aufweisen.

Der Wärmetauscher kann einen thermoelektrischen Generator 120 aufweisen, der dazu ausgebildet ist, mittels der Temperaturdifferenz zwischen Dialysat und Permeat Energie zum Betreiben des Sensors und/oder zum Auslesen des Sensors und/oder zum Betreiben der Anzeigeeinheit und/oder zum Steuern eines Aktors zum Schalten des Ventils und/oder zum Betreiben des Aktors und/oder zum Laden der Batterie bereitzustellen.

Das System kann optional eine Datenverbindung 121 zwischen dem Wärmetauscher und der Dialysemaschine aufweisen und dazu ausgebildet sein, Sensordaten, insbesondere Daten des Temperatursensors, von dem Wärmetauscher an die Dialysemaschine zu übertragen, insbesondere zum Regeln der Permeat-Temperatur.

In Figur 1 ist auch ein Ringleitungssystem 119 gezeigt, das optional Teil des Systems der vorliegenden Offenbarung sein kann. Der Wärmetauscher kann, bezogen auf die Zulaufrichtung von Permeat, zwischen dem Ringleitungssystem und der Dialysemaschine angeordnet sein. Insbesondere kann die Dialysemaschine im ordnungsgemäßen Betrieb über den Wärmetauscher mit dem Ringleitungssystem verbunden sein. Dabei umfasst das Ringleitungssystem beispielsweise eine Wasserleitung, die von einer Wasseraufbereitungsanlage (beispielsweise Umkehrosmoseanlage) kommt und Dialysemaschinen mit Frischwasser versorgt.

Weitere Merkmale und Vorteile sind im Folgenden beschrieben.

Die vorliegende Offenbarung betrifft ein System mit einer Dialysemaschine und einem Wärmetauscher, der im Folgenden auch als Rekuperator bezeichnet wird. Diese sind als unabhängige Einrichtungen ausgebildet. Daher wird der Rekuperator auch als externer Rekuperator bezeichnet. Der Wärmetauscher ist für eine Dialysemaschine, also ein Gerät zur extrakorporalen Blutbehandlung als externer Rekuperator vorgesehen, der zwischen Ringleitungssystem und Gerät, insbesondere nachrüstbar, installiert werden kann, um die Abwärme abfließender Flüssigkeiten zur Erwärmung zufließender Flüssigkeiten zu nutzen und somit Energie zu sparen.

Der Wärmetauscher der vorliegenden Offenbarung kann insbesondere kein Bestandteil der Dialysemaschine, kein Bestandteil des Ringleitungssystems und kein Bestandteil der Umkehrosmoseanlage sein.

Rekuperatoren und ihre Anwendung in Dialysemaschinen zum Vorheizen von hochreinem Dialysewasser (Permeat) sind bekannt. Der Rekuperator ist hierbei Teil der Maschine und im Hydrauliksystem verbaut. Im einfachsten Fall handelt es sich um eine Heizspirale, durch welche das abfließende Dialysat strömt und die sich in einem Vorlaufbehälter befindet. In den Vorlaufbehälter strömt das zu erwärmende Permeat, welches im Nachgang mit einer basischen und einer sauren Komponente versetzt wird. Einfache Heizspiralen haben allerdings den Nachteil, dass sie wenig effizient sind und nicht allzu viel Wärme zurückgewonnen werden kann. Alternativ kommen auch Platten-Rekuperatoren zum Einsatz, die mit einer größeren Austauschfläche einhergehen und somit den Effizienzgrad erhöhen. Nachteilig ist aber, dass Platten-Rekuperatoren wartungsintensiv und im eingebauten Zustand schwer bis kaum zu entleeren sind, was allerdings vor Auslieferung einer Dialysemaschine geschehen muss. In manchen Fällen sind Wärmetauscher (ggf. herausnehmbarer) Bestandteil der Dialysemaschine. Diese muss zur Entnahme aber geöffnet werden, was nur von Servicetechnikern durchgeführt werden kann). Generell gilt auch, dass der Wirkungsgrad mit steigender Austauschfläche zunimmt. Da aber der Bauraum innerhalb einer Dialysemaschine begrenzt ist, kann die Abwärme nicht immer effizient genutzt werden.

Würde man versuchen, stattdessen den Rekuperator als Teil des Ringleitungssystems auszulegen, wäre eine Integration des Rekuperators in ein bestehendes Ringleitungssystem mit einem hohen Aufwand verbunden oder gar nicht möglich sein.

Das System der vorliegenden Offenbarung ermöglicht, einen Rekuperator bereitzustellen, der die Abwärme des abfließenden Dialysats nutzt, um das zufließende Permeat vorzuwärmen. Der Rekuperator kann zwischen dem Ringleitungssystem und der Dialysemaschine eingebunden sein. Der Rekuperator ist somit kein Bestandteil der Maschine. Ebenfalls ist der Rekuperator optional kein Bestandteil des Ringleitungssystems. Die von der Dialysemaschine und gegebenenfalls Ringleitungssystem unabhängige Ausgestaltung ermöglicht, Bestandsmaschinen mit diesem externen Rekuperator leichter nachträglich ausrüsten zu können.

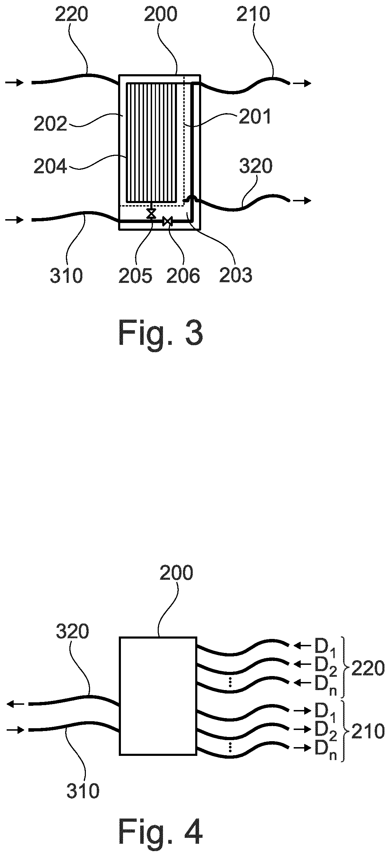

Somit ist der Wärmetauscher bei Bedarf anwendbar, ohne dafür Änderungen an der Maschine bzw. gegebenenfalls am Ringleitungssystem vornehmen zu müssen. Mehrere Maschinen sind an einem Wärmetauscher anschließbar (Figur 4) und mehrere Wärmetauscher in Reihe und/oder parallel sind möglich. Es gibt die Möglichkeit der passiven Ausführungsform mit stetiger Wärmeübertragung oder aktiven Ausführungsform, bei der temperaturabhängig aktiv die Ventilstellungen im Wärmetauscher eingestellt wird, beispielsweise Pfade freigegeben werden (Figur 3).

Figur 2 zeigt schematisch eine Dialysemaschine 10 gemäß der vorliegenden Offenbarung, an die ein Rekuperator 200 angeschlossen ist. Durch die Leitung 310 gelangt Permeat aus einem Ringleitungssystem oder aus einer Umkehrosmoseanlage in den Rekuperator 200 und anschließend durch die Leitung 210 weiter in die Dialysemaschine 10. Das (warme) Dialysat fließt durch die Leitung 220 in den Rekuperator 200 und dann durch die Leitung 320 in den Abfluss oder Aufbereitungseinheit. Die Flüssigkeiten werden dabei in entgegengesetzter Richtung (Gegenstromprinzip) aneinander vorbeigeführt. Im Rekuperator 200 findet der Wärmeaustausch zwischen Dialysat und Permeat statt. Als Rekuperator 200 kommen verschiedene Bauweisen in Frage. Darunter fallen insbesondere Doppelrohr-, Rohrbündel- und Platten-Rekuperatoren. Da sich der Rekuperator 200 außerhalb der Dialysemaschine 10 befindet, ist eine limitierende Beachtung der Größe nicht zwingend erforderlich, da genügend Platz außerhalb der Maschine vorhanden ist. Als korrosionsbeständiges Material der Wärmeübertragungseinheit kann Edelstahl, welcher eine gute Wärmeleitfähigkeit und eine hohe Robustheit aufweist, verwendet werden. Alternativ sind polymerbasierte Einheiten denkbar. Sie bieten den Vorteil, dass nahezu beliebige Strukturen insbesondere durch additive Fertigungsverfahren umsetzbar sind und die Austauschoberfläche sowie das Strömungsverhalten somit optimiert werden können. Als Materialien kommen z.B. wärmeleitfähiges Polypropylen oder Polyphenylensulfid in Frage. Durch das im Vergleich zu Edelstahl geringere Gewicht können die polymerbasierten Rekuperatoren zudem leichter transportiert werden. Der Rekuperator 200 kann auf den Boden gestellt werden oder Rollen aufweisen, sodass er bei Bedarf geschoben werden kann. Außerdem ist es möglich, den Rekuperator mit einem Element zum Aufhängen an einer Dialysemaschine 10 auszustatten. Es bietet sich an, die Distanz zwischen Rekuperator 200 und Dialysemaschine 10 und somit die Länge der Leitungen 210 und 220 so kurz wie möglich zu wählen, um Wärmeverluste zu vermeiden. Auch können die Leitungen 210 und 220 wärmeisoliert ausgeführt sein. Zur Reinigung bzw. Desinfektion oder Entkalkung kann die Wärmeübertragungseinheit über eine Stelle zum Applizieren eines Reinigungsmittels aufweisen, welches dann die Strömungswege durchspült. Die vorliegende Offenbarung umfasst auch die Möglichkeit, mehrere Rekuperatoren hintereinander zu schalten, um die Effizienz zu steigern.

Es ist möglich, den Rekuperator neben der eigentlichen Wärmeübertragungseinheit mit weiteren Elementen, d.h. insbesondere Sensoren und Aktoren, auszustatten. Figur 3 zeigt den Rekuperator 200 aus Figur 2 beispielhaft in einer solchen Ausführung. Eine Wand 201 teilt das Innere des Rekuperators 200 in zwei Räume 202 und 203, wobei sich in Raum 202 die eigentliche Wärmeübertragungseinheit 204 befindet. In Raum 203 befinden sich die Ventile 205 und 206, durch welche das zu erwärmende Permeat aus Leitung 310 entweder in die Wärmeübertragungseinheit 204 oder direkt in die Leitung 210 gelangt. Letzteres umgeht eine Erwärmung des Permeats, was insbesondere nach einer Desinfektion von Vorteil ist, da bei einer Desinfektion heiße Flüssigkeit durch die Leitung 220 abfließt und frisches Permeat in manchen Fällen (beispielsweise, wenn eine neue Dialysetherapie vorbereitet werden soll) nur unnötig (über eine physiologisch verträgliche Temperatur) aufgeheizt werden würde. Der Rekuperator 200 kann hierfür auch mindestens einen Temperatursensor aufweisen, der mindestens die Temperatur des von der Dialysemaschine 10 abfließenden Dialysats misst. Wird ein Schwellwert (z.B. 40°C) überschritten, kann das Ventil 205 schließen und das Ventil 206 öffnen, um einer unerwünschten Erwärmung des Permeats entgegenzuwirken. Auch kann die Temperatur genutzt werden, um das automatische Applizieren eines Reinigungsmittels in die Strömungswege des Rekuperators temperaturabhängig zu steuern, da die Reinigungseffizienz mit steigender Temperatur zunimmt.

Der Rekuperator 200 kann außerdem über eine Anzeigeeinheit verfügen, die zum Beispiel die Ventilstellung und/oder Temperaturen und/oder weitere Sensordaten anzeigt. Die Energieversorgung kann hierfür über eine Batterie erfolgen oder vom Netz bereitgestellt werden. Darüber hinaus ist es möglich, den Rekuperator energieautark zu gestalten. Hierfür kann bspw. der Seebeck- oder Peltier-Effekt genutzt werden, um anhand der Temperaturdifferenz zwischen dem warmen Dialysat und dem kälteren Permeat eine Spannung zu erzeugen, die dann zum Auslesen von Sensorwerten, zum Ansteuern von Aktorik und/oder zum Betreiben der Anzeigeeinheit genutzt wird. Des Weiteren ist eine kabellose oder kabelgebundene Verbindung mit einer Dialysemaschine möglich. Es ist zum Beispiel denkbar, die Temperaturdaten des Rekuperators an die angeschlossene Maschine zu übertragen, um mit diesen Daten die Regelung der Dialysierflüssigkeitstemperatur zu optimieren. Die vorliegende Offenbarung sieht optional vor, einen Rekuperator für mehrere Maschinen einzusetzen. Hierfür weist der Rekuperator 200 mehrere Permeat-Leitungen 210 auf, die zu den Dialysemaschinen D₁ bis Dₙ führen, sowie Dialysat-Leitungen 220, die von den Maschinen D₁ bis Dₙ zum Rekuperator 200 führen (siehe beispielsweise Figur 4).

Auch dieser Rekuperator ist zwischen einem herkömmlichen Ringleitungssystem und Dialysemaschinen nachträglich integrierbar.

Obwohl die Erfindung in den Zeichnungen und der vorstehenden Beschreibung detailliert dargestellt und beschrieben ist, sind diese Darstellungen und Beschreibungen als beispielhaft und nicht einschränkend zu betrachten. Die Erfindung ist nicht auf die offengelegten Ausführungsformen beschränkt. In Anbetracht der vorstehenden Beschreibung und der Zeichnungen wird es für den Fachmann offensichtlich sein, dass im Rahmen der Erfindung, wie sie in den Ansprüchen definiert ist, verschiedene Modifikationen vorgenommen werden können.

## Patentansprüche

1. System (100) umfassend eine Vorrichtung zur extrakorporalen Blutbehandlung (101) und einen Wärmetauscher (102) zum Wärmeaustausch zwischen von der Vorrichtung zur extrakorporalen Blutbehandlung (101) abfließendem Dialysat und der Vorrichtung zur extrakorporalen Blutbehandlung (101) zuzuführendem Permeat, wobei die Vorrichtung zur extrakorporalen Blutbehandlung (101) und der Wärmetauscher (102) als separate Einrichtungen ausgebildet sind.

2. System (100) nach Anspruch 1,
wobei die Vorrichtung zur extrakorporalen Blutbehandlung (101) und der Wärmetauscher (102) über einen ersten Anschluss (103a) der Vorrichtung zur extrakorporalen Blutbehandlung (101), einen zweiten Anschluss (103b) der Vorrichtung zur extrakorporalen Blutbehandlung (101), einen ersten Anschluss (104a) des Wärmetauschers (102) und einen zweiten Anschluss (104b) des Wärmetauschers (102) miteinander verbunden bzw. verbindbar sind und wobei das System (100) eine erste Leitung (105a), beispielsweise einen ersten Schlauch, und eine zweite Leitung (105b), beispielsweise einen zweiten Schlauch, umfasst und der erste Anschluss (104a) des Wärmetauschers (102) mittels der ersten Leitung (105a) mit dem ersten Anschluss (103a) der Vorrichtung zur extrakorporalen Blutbehandlung (101) verbunden ist und der zweite Anschluss (104b) des Wärmetauschers(102) mittels der zweiten Leitung (105b) mit dem zweiten Anschluss (103b) der Vorrichtung zur extrakorporalen Blutbehandlung (101) verbunden ist, insbesondere wobei die erste Leitung (105a) und/oder die zweite Leitung (105b) wärmeisoliert ist/sind, oder
wobei die Vorrichtung zur extrakorporalen Blutbehandlung (101) und der Wärmetauscher (102) über einen ersten Anschluss (103a) der Vorrichtung zur extrakorporalen Blutbehandlung (101), einen zweiten Anschluss (103b) der Vorrichtung zur extrakorporalen Blutbehandlung (101), einen ersten Anschluss (104a) des Wärmetauschers (102) und einen zweiten Anschluss (104b) des Wärmetauschers (102) miteinander verbunden bzw. verbindbar sind.

3. System (100) nach einem der vorangegangenen Ansprüche,
wobei der Wärmetauscher (102) einen Wärmeübertragungsabschnitt (106), mindestens ein mittels eines Aktors (107) schaltbares Ventil (108) und einen Sensor (109) umfasst, insbesondere einen Temperatursensor,
wobei der Wärmetauscher (102) dazu ausgebildet ist, das Ventil (108) mittels des Aktors (107) basierend auf Sensordaten des Sensors (109) zu schalten, insbesondere derart zu schalten, dass bei Feststellen eines Überschreitens eines ersten Temperaturschwellwerts einer von der Vorrichtung zur extrakorporalen Blutbehandlung durch den Wärmetauscher abfließenden Flüssigkeit, beispielsweise des Dialysats oder einer bei einer Desinfektion, Entkalkung, und/oder Reinigung der Vorrichtung zur extrakorporalen Blutbehandlung verwendeten Flüssigkeit, mittels des Sensors (109) das mindestens eine Ventil (108) derart geschaltet wird, dass das Permeat und/oder die abfließende Flüssigkeit nicht durch den Wärmeübertragungsabschnitt (106) fließt.

4. System (100) nach einem der vorangegangenen Ansprüche,
wobei der Wärmetauscher (102) einen/den Temperatursensor umfasst, wobei der Wärmetauscher (102) dazu ausgebildet ist, bei Überschreiten eines zweiten Temperaturschwellwerts einer von der Vorrichtung zur extrakorporalen Blutbehandlung durch den Wärmetauscher abfließenden Flüssigkeit, beispielsweise des Dialysats oder einer bei einer Desinfektion, Entkalkung, und/oder Reinigung der Vorrichtung zur extrakorporalen Blutbehandlung verwendeten Flüssigkeit, automatisch eine Wartungsflüssigkeit, beispielsweise Reinigungsmittel, Desinfektionsmittel oder Entkalkungsmittel, in Strömungswege des Wärmetauschers (102) zuzuführen, insbesondere in einen/den Wärmeübertragungsabschnitt (106) des Wärmetauschers (102).

5. System (100) nach einem der vorangegangenen Ansprüche,
wobei der Wärmetauscher (102) einen Wartungsflüssigkeitszufuhrabschnitt (110) aufweist, über den eine/die Wartungsflüssigkeit, beispielsweise Reinigungsmittel, Desinfektionsmittel oder Entkalkungsmittel, in Strömungswege des Wärmetauschers (102) zuführbar ist, insbesondere in einen/den Wärmeübertragungsabschnitt des (106) Wärmetauschers (102).

6. System (100) nach einem der vorangegangenen Ansprüche,
wobei das System (100) mehrere Vorrichtungen zur extrakorporalen Blutbehandlung (101, 111a, 111b, 111c) umfasst und der Wärmetauscher (102) mit den Vorrichtungen zur extrakorporalen Blutbehandlung (101, 111a, 111b, 111c) über deren jeweiligen ersten Anschluss (103a) und zweiten Anschluss (103b) verbunden ist, und/oder
wobei das System (100) mehrere der Wärmetauscher (102, 112a, 112b, 112c, 112d) umfasst, insbesondere mehrere bezogen auf die Strömungsrichtung in Reihe geschaltete Wärmetauscher.

7. System (100) nach einem der vorangegangenen Ansprüche,
wobei der Wärmetauscher (102) als Gegenstromwärmetauscher ausgebildet ist, und/oder
wobei der Wärmetauscher (102) als Doppelrohrrekuperator, Rohrbündelrekuperator oder Plattenrekuperator ausgebildet ist, und/oder
wobei der/ein Wärmeübertragungsabschnitt (106) des Wärmetauschers (102) aus Edelstahl oder polymerbasiertem Material, insbesondere Polypropylen oder Polyphenylensulfid, ausgebildet ist.

8. System (100) nach einem der vorangegangenen Ansprüche,
wobei der Wärmetauscher (102) im ordnungsgemäßen Betrieb auf dem Boden steht, und/oder
wobei der Wärmetauscher (102) Rollen (113) aufweist, mittels derer der Wärmetauscher (102) transportierbar ist, insbesondere auf denen der Wärmetauscher (102) beim Transport und optional im ordnungsgemäßen Betrieb gelagert ist, und/oder
wobei das System (100) ein Hängesystem (114) umfasst, das zum Aufhängen des Wärmetauschers (112d) an der Vorrichtung zur extrakorporalen Blutbehandlung (101), insbesondere an einer Außenseite eines Maschinengehäuses (115) der Vorrichtung zur extrakorporalen Blutbehandlung (101), ausgebildet ist.

9. System (100) nach einem der vorangegangenen Ansprüche,
wobei der Wärmetauscher (102), bezogen auf die Zulaufrichtung von Permeat, stromaufwärts eines Zulaufventils (101a) zum Dialysierflüssigkeitskreislaufs (101b) der Vorrichtung zur extrakorporalen Blutbehandlung (101) angeordnet ist, und/oder
wobei der Wärmetauscher (102), bezogen auf die Zulaufrichtung von Permeat, zwischen einem Ringleitungssystem (119) und der Vorrichtung zur extrakorporalen Blutbehandlung (101) angeordnet ist, insbesondere wobei das System (100) ein Ringleitungssystem (119) umfasst und die Vorrichtung zur extrakorporalen Blutbehandlung (101) im ordnungsgemäßen Betrieb über den Wärmetauscher (102) mit dem Ringleitungssystem (119) verbunden ist.

10. System (100) nach einem der Ansprüche 1 bis 9,
wobei die Vorrichtung zur extrakorporalen Blutbehandlung (101) ein Maschinengehäuse (115) aufweist, insbesondere an dessen Außenseite ein/der erste Anschluss (103a) und ein/der zweite Anschluss (103b) der Vorrichtung zur extrakorporalen Blutbehandlung (101) angeordnet sind, und/oder
wobei der Wärmetauscher (102) ein Gehäuse (116) aufweist, insbesondere an dessen Außenseite ein/der erste Anschluss (104a) und ein/der zweite Anschluss (104b) des Wärmetauschers (102) angeordnet sind.

11. System (100) nach Anspruch 10,
wobei die Vorrichtung zur extrakorporalen Blutbehandlung (101) das Maschinengehäuse (115) aufweist und der Wärmetauscher (102) außerhalb des Maschinengehäuses (115) angeordnet ist, und/oder
wobei der Wärmetauscher (102) das Gehäuse (116) aufweist und die Vorrichtung zur extrakorporalen Blutbehandlung (101) das Maschinengehäuse (115) aufweist, wobei das Gehäuse (116) des Wärmetauschers (102) außerhalb des Maschinengehäuses (115) der Vorrichtung zur extrakorporalen Blutbehandlung (101) angeordnet ist.

12. System (100) nach einem der vorangegangenen Ansprüche,
wobei der Wärmetauscher (102) an die Vorrichtung zur extrakorporalen Blutbehandlung (101) derart angeschlossen ist, dass im ordnungsgemäßen Betrieb im Wärmetauscher (102), insbesondere in einem/dem Wärmeübertragungsabschnitt (106) des Wärmetauschers (102), ein Wärmeaustausch zwischen vom Wärmetauscher (102) an die Vorrichtung zur extrakorporalen Blutbehandlung (101) bereitzustellenden Permeat und aus der Vorrichtung zur extrakorporalen Blutbehandlung (101) abfließenden Dialysat erfolgt.

13. System (100) nach einem der vorangegangenen Ansprüche,
wobei das System (100), insbesondere der Wärmetauscher (102), eine Anzeigeeinheit (117) aufweist, die zum Anzeigen einer Ventilstellung eines/des Ventils (108) und/oder von Sensordaten eines/des Sensors (109), insbesondere einer Temperatur einer von der Vorrichtung zur extrakorporalen Blutbehandlung abfließenden Flüssigkeit, insbesondere des Dialysats, ausgebildet ist, und/oder
wobei das System (100), insbesondere der Wärmetauscher (102), eine Energieversorgung (118), insbesondere eine Batterie und/oder Netzstromversorgung, zum Betreiben des Sensors (109) und/oder zum Auslesen des Sensors (109) und/oder zum Betreiben der Anzeigeeinheit (117) und/oder zum Steuern eines/des Aktors (107) zum Schalten des Ventils (108) und/oder zum Betreiben des Aktors (107) aufweist, und/oder
wobei der Wärmetauscher (102) einen thermoelektrischen Generator (120) aufweist, der dazu ausgebildet ist, mittels der Temperaturdifferenz zwischen einer von der Vorrichtung zur extrakorporalen Blutbehandlung abfließenden Flüssigkeit, insbesondere dem Dialysat, und Permeat Energie zum Betreiben des Sensors (109) und/oder zum Auslesen des Sensors (109) und/oder zum Betreiben der Anzeigeeinheit (117) und/oder zum Steuern eines/des Aktors (107) zum Schalten des Ventils (108) und/oder zum Betreiben des Aktors (107) und/oder zum Laden der Batterie bereitzustellen.

14. System (100) nach einem der vorangegangenen Ansprüche, wobei das System (100) eine Datenverbindung (121) zwischen dem Wärmetauscher (102) und der Vorrichtung zur extrakorporalen Blutbehandlung (101) aufweist und dazu ausgebildet ist, Sensordaten, insbesondere Daten eines Temperatursensors, von dem Wärmetauscher (102) an die Vorrichtung zur extrakorporalen Blutbehandlung (101) zu übertragen, insbesondere zum Regeln der Permeat-Temperatur, wobei optional eine Datenverbindung und eine Energieversorgung mittels eines gemeinsamen Verbindungselements bereit gestellt sind, beispielsweise mittels eines Verbindungskabels, das Energieversorgung und Datentransfer erlaubt.

15. Wärmetauscher (102) zur Verwendung in einem System (100) gemäß einem der vorangegangenen Ansprüche, insbesondere zum Verbinden mit einer oder mehreren Vorrichtungen zur extrakorporalen Blutbehandlung (101) gemäß den vorangegangenen Ansprüchen.
